# EUROPEAN PATENT APPLICATION

(11) **EP 4 481 686 A1**
(43) Date of publication of application: **25.12.2024**
(21) Application number: 24180059.8
(22) Date of filing: 04.06.2024
(51) Int. Cl.: G06T 11/00

(54) **METHOD, SYSTEM AND/OR COMPUTER READABLE MEDIUM FOR LOCAL MOTION CORRECTION BASED ON PET DATA**

(30) Priority: 23.06.2023 US 202318213432
(71) Applicant: GE Precision Healthcare LLC, Waukesha, WI 53188 (US)
(72) Inventor: SPANGLER-BICKELL, Matthew Gilbert, Cambria, 93428 (US); HEUKENSFELDT JANSEN, Floribertus Philippus Martinus, Ballston Lake, 12019 (US); DELLER, Timothy Wayne, Brookfield, 53005 (US)
(74) Representative: Kilburn & Strode LLP

(57) **Abstract**

A computer-implemented method includes obtaining positron emission tomography (PET) data that includes moving tissue of interest and generating a set of short PET frames from the PET data, wherein each short PET frame of the set of short PET frames is based on a time duration. The computer-implemented method further includes identifying a first tissue of interest in the set of short PET frames and identifying at least a second tissue of interest in the set of short PET frames. The computer-implemented method further includes estimating, separately and independently, a first motion of the first tissue of interest and a second motion of second tissue of interest based on the short PET frames and motion correcting, separately and independently, the first tissue of interest for the first motion and the second tissue of interest for the second motion.

## Description

### FIELD

The following generally relates to Positron Emission Tomography (PET) and more particularly to local motion correction based on PET data.

### BACKGROUND

Positron Emission Tomography (PET) is a functional imaging modality that utilizes a radiopharmaceutical with a tissue targeted radionuclide (i.e., a radiotracer) to visualize and/or measure functional processes such as metabolism, blood flow, absorption, etc. Prior to a PET scan, a radiopharmaceutical is administered to a patient. As the radionuclide accumulates within organs, vessels, or the like, the radionuclide undergoes positron emission decay and emits a positron. When the positron collides with an electron in the surrounding tissue, both the positron and the electron are annihilated and converted into a pair of photons, or gamma rays, each having an energy of 511 keV.

The two photons are directed in substantially opposite directions along a line of response (LOR) and are coincidently detected when they reach respective detectors positioned across from each other on a detector ring assembly, approximately one hundred and eighty degrees apart from each other. When the photons impinge upon scintillation crystals of the detectors, a scintillation event (e.g., flash of light) is produced for each event, and detectors detect the scintillation events and produce electrical signals indicative thereof. The electrical signals are processed to generate PET data, which represent a distribution of the radiopharmaceutical within the patient, which may be employed to observe metabolic processes, etc. in the body and diagnose disease.

A PET scan can take several minutes, e.g., 8-10 minutes. As such, the acquired data may reflect moving tissue. Sources of such movement include periodic motion such as breathing, the heart beating, etc., and/or non-periodic motion such as the patient coughing, moving, etc. Unfortunately, motion can manifest as a visible artifact such as blur in the PET data. Existing approaches to mitigate blur in PET data due to motion generally also tend to degrade image quality such as reduce the signal-to-noise ratio. In view of at least the foregoing, there is an unresolved need for an improved approach(es) to mitigate motion related artifact in PET data.

### SUMMARY

Aspects described herein address the above-referenced problems and others. This summary introduces concepts that are described in more detail in the detailed description. It should not be used to identify essential features of the claimed subject matter, nor to limit the scope of the claimed subject matter.

This section will include the independent claims written in paragraph form. I will complete this section once the claims are approved.

In one aspect, a computer-implemented method includes obtaining positron emission tomography (PET) data that includes moving tissue of interest. The computer-implemented method further includes generating a set of short PET frames from the PET data, wherein each short PET frame of the set of short PET frames is based on a time duration. The computer-implemented method further includes identifying a first tissue of interest in the set of short PET frames. The computer-implemented method further includes identifying at least a second tissue of interest in the set of short PET frames. The computer-implemented method further includes estimating, separately and independently, a first motion of the first tissue of interest and a second motion of second tissue of interest based on the short PET frames. The computer-implemented method further includes motion correcting, separately and independently, the first tissue of interest for the first motion and the second tissue of interest for the second motion.

In another aspect, a computer system includes a computer readable storage medium with instructions for correcting motion in data and a processor configured to execute the instructions. The instructions cause the processor to obtain PET data that includes moving tissue of interest, generate a set of short PET frames from the PET data, wherein each short PET frame of the set of short PET frames is based on a time duration, identify a first tissue of interest in the set of short PET frames, identify at least a second tissue of interest in set of short PET frames, estimate, separately and independently, a first motion of the first tissue of interest and a second motion of second tissue of interest based on the short PET frames, and motion correct, separately and independently, the first tissue of interest for the first motion and the second tissue of interest for the second motion.

In another aspect, a computer readable medium is encoded with computer executable instructions, which, when executed by a processor, cause the processor to obtain PET data that includes moving tissue of interest, generate a set of short PET frames from the PET data, wherein each short PET frame of the set of short PET frames is based on a time duration, identify a first tissue of interest in the set of short PET frames, identify at least a second tissue of interest in set of short PET frames, estimate, separately and independently, a first motion of the first tissue of interest and a second motion of second tissue of interest based on the short PET frames, and motion correct, separately and independently, the first tissue of interest for the first motion and the second tissue of interest for the second motion.

Those skilled in the art will recognize still other aspects of the present application upon reading and understanding the attached description.

### BRIEF DESCRIPTION OF THE DRAWINGS

The application is illustrated by way of example and not limited by the figures of the accompanying drawings in which like references indicate similar elements.
FIGURE 1 schematically illustrates a cross-sectional side view of a non-limiting example of an imaging system, in accordance with an embodiment(s) herein.
FIGURE 2 schematically illustrates a non-limiting example of the imaging system configured for PET imaging, in accordance with an embodiment(s) herein.
FIGURE 3 schematically illustrates a non-limiting example of the imaging system further configured for CT imaging, in accordance with an embodiment(s) herein.
FIGURE 4 schematically illustrates a non-limiting example of the console of the imaging system, in accordance with an embodiment(s) herein.
FIGURE 5 schematically illustrates a non-limiting example of a short frame generation module, in accordance with an embodiment(s) herein.
FIGURE 6 schematically illustrates a non-limiting example of a tissue of interest module, in accordance with an embodiment(s) herein.
FIGURE 7 graphically illustrates a PET MIP rendering, in accordance with an embodiment(s) herein.
FIGURE 8 graphically illustrates a sub-portion of the PET MIP rendering of FIGURE 7, in accordance with an embodiment(s) herein.
FIGURE 9 graphically illustrates selection of a tissue of interest in the sub-portion of the PET MIP rendering of FIGURE 7, in accordance with an embodiment(s) herein.
FIGURE 10 graphically illustrates selection of another tissue of interest in the sub-portion of the PET MIP rendering of FIGURE 7, in accordance with an embodiment(s) herein.
FIGURE 11 graphically illustrates the selected tissues of interest bounded by regions of interest, in accordance with an embodiment(s) herein.
FIGURE 12 graphically illustrates a non-limiting display of PET data for assisting with defining the regions of interest, in accordance with an embodiment(s) herein.
FIGURE 13 schematically illustrates a non-limiting example of a motion correction of local patches module, in accordance with an embodiment(s) herein.
FIGURE 14 graphically illustrates example motion in a volume of interest in the x direction, in accordance with an embodiment(s) herein.
FIGURE 15 graphically illustrates example motion in the volume of interest in the y direction, in accordance with an embodiment(s) herein.
FIGURE 16 graphically illustrates example motion in the volume of interest in the z direction, in accordance with an embodiment(s) herein.
FIGURE 17 schematically illustrates a non-limiting example of a local motion compensation module, in accordance with an embodiment(s) herein.
FIGURE 18 graphically illustrates display of a motion corrected first patch, in accordance with an embodiment(s) herein.
FIGURE 19 graphically illustrates display of a motion corrected second patch, in accordance with an embodiment(s) herein.
FIGURE 20 graphically illustrates display of a motion corrected third patch, in accordance with an embodiment(s) herein.
FIGURE 21 graphically illustrates display of a motion corrected fourth patch, in accordance with an embodiment(s) herein.
FIGURE 22 graphically illustrates display of a sub-portion of the PET MIP rendering with the motion corrected patches, in accordance with an embodiment(s) herein.
FIGURE 23 schematically illustrates a variation of FIGURE 1 with an imaging sub-system configured for MR imaging, in accordance with an embodiment(s) herein.
FIGURE 24 illustrates a non-limiting example of a flow chart for a computer-implemented method, in accordance with an embodiment(s) herein.

### DETAILED DESCRIPTION

Positron Emission Tomography (PET) is a functional imaging modality that utilizes a radiopharmaceutical that includes a tissue targeted radionuclide (i.e., a radiotracer) to visualize and measure functional processes such as metabolism. The radionuclide may include fluorine-18, carbon-11, nitrogen-13, oxygen-15, etc. A non-limiting example of such a radiopharmaceutical includes F-18 fluorodeoxyglucose (FDG), which includes a glucose analog with the positron-emitting radionuclide fluorine-18 substituted for the normal hydroxyl group at the C-2 position in the glucose molecule. The uptake of FDG by tissues is a marker for the tissue uptake of glucose, which is correlated with certain types of tissue metabolism.

For a PET scan, a prescribed radiopharmaceutical is first administered to a patient. As the radiopharmaceutical accumulates within organs, vessels, or the like, the radionuclide undergoes positron emission decay and emits a positron. When the positron collides with an electron in the surrounding tissue, both the positron and the electron are annihilated and converted into a pair of photons, or gamma rays, each having an energy of 511 keV. The two photons are directed in substantially opposite directions along a line of response (LOR) and are coincidently detected when they reach respective detectors positioned across from each other on a detector ring assembly, approximately one hundred and eighty degrees apart from each other. The detectors produce electrical signals indicative thereof.

The electrical signals are processed to generate PET data, which represent a distribution of the radiopharmaceutical within the patient, which may be employed to observe metabolic processes, etc. in the body and diagnose disease. In general, PET scan data acquisition takes several minutes, e.g., 8-10 minutes, and the acquired data may include moving tissue, which manifests as a visible artifact such as blur in the PET data, which may degrade image quality. Sources of such movement include periodic motion such as breathing, the heart beating, etc., and/or non-periodic motion such as the patient coughing, moving, etc. Unfortunately, existing approaches to mitigate blur in PET data due to motion tend to degrade image quality, e.g., by reducing the signal-to-noise ratio.

Described herein is an approach(es) that mitigates motion artifact and, hence, the blur. The approach(es), in general, includes generating a series of short PET frames spanning a PET acquisition from the acquired PET data, identifying at least two tissues of interest in a rendering of the PET data, separately and independently estimating a motion of the at least two tissues of interest, and generating motion corrected patches of the at least two tissues of interest based on the estimated motions. In one instance, the individual motion corrected patches are displayed. Additionally, or alternatively, the individual motion corrected patches are inserted into a rendering of the PET data at corresponding locations, and the rendering of the PET data is displayed.

In one instance, since each volume of interest is local to a corresponding tissue of interest, the motion therein of each tissue of interest generally moves rigidly due to periodic (e.g., respiration, etc.) and/or non-period motion (e.g., bulk patient movement), and straight line projectors are processed. In another instance, the motion includes affine motion, allowing for shear and scale, again using straight line projectors. The short PET frames are utilized to estimate the rigid motion of each tissue of interest in a local volume of interest using a known (e.g., registration, maximum pixel, center-of-mass, etc.) and/or other motion estimation approach(es). The tissue of interest in the local volume of interest has a strong signal that can be readily tracked. Tissues in other regions may have weak signals that may not be able to be readily tracked, which means that local regions of interest cannot be placed everywhere. In addition, a full FOV of the PET data will further include tissues that are moving differently, and, thus, a rigid motion cannot be assumed.

A full motion corrected reconstruction of a patch surrounding tissue of interest for each tissue of interest moves each measured event to undo the estimated rigid motion, providing a quantitatively accurate patch. Motion correcting patches instead of the entire PET data will require less resources (e.g., processing power and/or memory) at least since the aggregate of the number of events in the patches will be lower than the number of total events in the PET data. Embodiments will now be described, by way of example, with reference to the Figures.

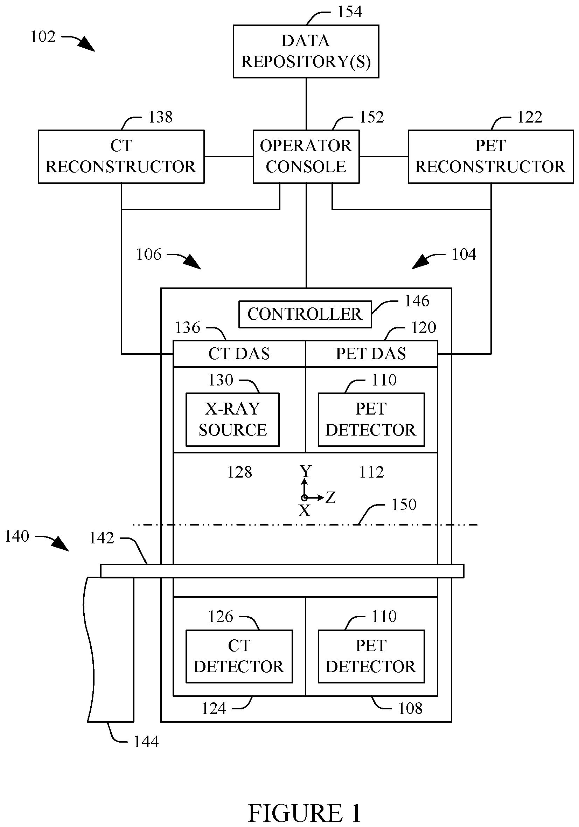

FIGURE 1 schematically illustrates a cross-sectional side view of a non-limiting example of an imaging system 102. In one instance, the imaging system 102 includes a first imaging sub-system 104 and a second imaging sub-system 106. The first imaging sub-system 104 is configured for PET imaging, and the second imaging sub-system 106 is configured for Computerized Tomography (CT) imaging. In another instance, the first imaging sub-system 104 is configured for CT imaging, and the second imaging sub-system 106 is configured for PET imaging. In yet another instance, the first imaging sub-system 104 and the second imaging sub-system 106 are separate imaging systems.

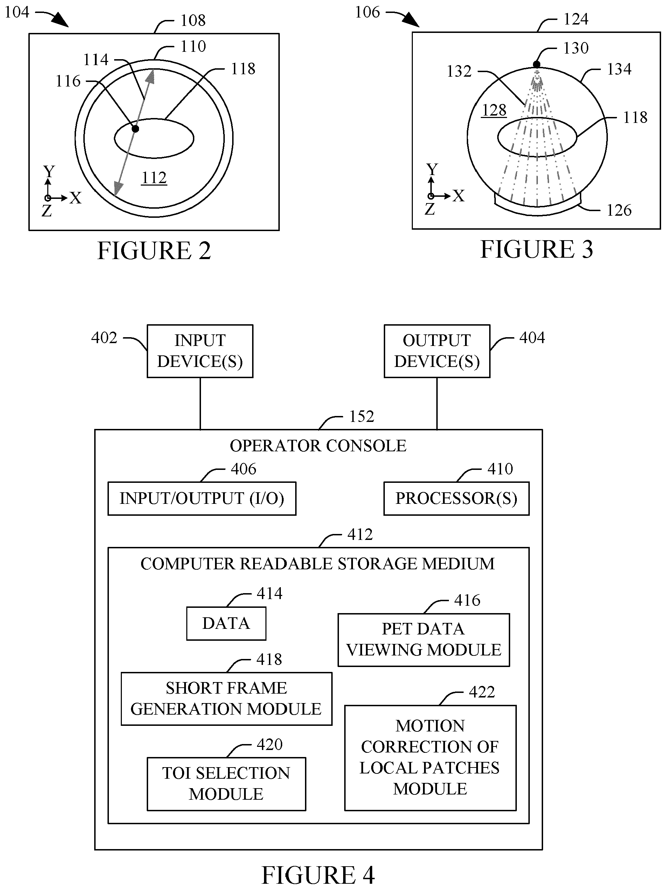

FIGURE 2 schematically illustrates a front view of the first imaging sub-system 104 configured for PET imaging. With reference to FIGURES 1 and 2, the first imaging sub-system 104 includes a PET gantry 108. The PET gantry 108 includes a radiation sensitive detector array 110 disposed about a PET examination region 112 in a generally annular ring. The radiation sensitive detector array 110 includes a plurality of detectors (photosensors) in optical communication with a scintillator material (scintillation crystals), which is disposed between the plurality of detectors and the PET examination region 112.

The scintillator material converts 511 keV gamma radiation 114 (FIGURE 2) produced in response to a positron annihilation event 116 (FIGURE 2) occurring in the examination region 112 in a patient 118 (FIGURE 2) disposed therein into light photons, and the plurality of detectors convert the light photons into electrical signals. The plurality of detectors includes one or more photosensors, such as avalanche photodiodes, photomultipliers, silicon photomultipliers, and/or another type of photosensor.

The first imaging sub-system 104 further includes a PET data acquisition system (DAS) 120. The PET data acquisition system 120 receives data from the radiation sensitive detector array 110 and produces PET data, which includes a list of events detected by the plurality of radiation sensitive detectors 110. The PET data acquisition system 120 identifies coincident gamma pairs by identifying events detected in temporal coincidence (or near simultaneously) along a line of response (LOR), which is a straight line joining the two detectors detecting the events, and generates list mode data and/or a histogram (sinogram) indicative thereof.

Coincidence can be determined by a number of factors, including event time markers, which must be within a predetermined time period of each other to indicate coincidence, and the LOR. Events that cannot be paired can be discarded. Events that can be paired are located and recorded as a coincidence event pairs. The PET projection data provides information on the LOR for each event, such as a transverse position and a longitudinal position of the LOR and a transverse angle and an azimuthal angle.

Where the first imaging sub-system 104 is configured for time of flight (TOF), the PET projection data may also include TOF information, which allows a location of an event along a LOR to be estimated. For example, when a positron annihilation event occurs closer to a first detector crystal than a second detector crystal, one annihilation photon may reach the first detector crystal before (e.g., nanoseconds or picoseconds before) the other annihilation photon reaches the second detector crystal. The TOF difference may be used to constrain a location of the positron annihilation event along the LOR.

Additionally, or alternatively, the PET projection data is re-binned into one or more sinograms or projection bins. A PET reconstructor 122 reconstructs the PET projection data using known iterative or other techniques to generate volumetric image data (i.e., PET image data) indicative of the distribution of the radionuclide in a scanned object. The PET image data can be co-registered with CT image data, and the CT image data can be utilized to generate an attenuation map for attenuation and/or other desired corrections to the PET image data.

FIGURE 3 schematically illustrates a non-limiting example of a front view of the second imaging sub-system 104 configured for CT imaging. With reference to FIGURES 1 and 3, the second imaging sub-system 106 includes a CT gantry 124. The CT gantry 124 includes a radiation sensitive detector array 126 disposed about a CT examination region 128 in an annular ring. The CT gantry 124 further includes a radiation source 130, such as an X-ray tube or source, that rotates about the CT examination region 128. The radiation sensitive detector 126 detects radiation 132 (FIGURE 3) emitted by the radiation source 130 that has traversed the examination region 128 and the subject 118 (FIGURE 3) therein.

The radiation source 130 and the radiation sensitive detector array 126 are disposed on a rotating frame 134 (FIGURE 3), opposite each other, across the CT examination region 128. The rotating frame 134 rotates the X-ray source 130 in coordination with the array of X-ray radiation detectors 126. The X-ray source 130 emits the X-ray radiation 132 that traverses the examination region 128 and the subject 118 disposed therein, and the array of X-ray radiation detectors 126 detect X-ray radiation impingent thereon. For each arc segment, the array of X-ray radiation detectors 126 generates a view of projections.

A CT data acquisition system (DAS) 136 processes the signals from the CT detector 126 to generate data indicative of the radiation attenuation along a plurality of lines or rays through the examination region 128. A CT reconstructor 138 reconstructs the data using reconstruction algorithms to generate volumetric image data (i.e., CT image data) indicative of the radiation attenuation of the patient 116. Suitable reconstruction algorithms include an analytic image reconstruction algorithm such as filtered backprojection (FBP), etc., an iterative reconstruction algorithm such as advanced statistical iterative reconstruction (ASIR), a maximum likelihood expectation maximization (MLEM) algorithm, another algorithm and/or a combination thereof.

With reference to FIGURE 1, a subject support 140 includes a tabletop 142 moveably coupled to a frame/base 144. In one instance, the tabletop 142 is slidably coupled to the frame/base 144 via a bearing or the like, and a drive system (not visible) including a controller, a motor, a lead screw, and a nut (or other drive system) translates the tabletop 142 along the frame/base 144 into and out of the examination region 128 and/or 112. The tabletop 142 is configured to support an object or subject in the examination region 128 and/or 112 for loading, scanning, and/or unloading the subject or object.

A controller 146 is configured to control components such as rotation of the gantry 134, an operation of the X-ray source 130, an operation of the detector arrays 126 and/or 110, an operation of the subject support 140, etc. For example, in one embodiment the controller 146 includes an X-ray controller configured to provide power and timing signals to the X-ray source 130. In another example, the controller 146 includes a gantry motor controller configured to control a rotational speed and/or position of the gantry 134 based on imaging requirements. In yet another example, the controller 146 includes a subject support controller configured to control motion and/or height of the subject support 140 for loading, scanning and/or unloading the patient 116. Where the first imaging sub-system 104 and the second imaging sub-system 106 are separate imaging systems, each of the sub-systems 104 and 106 will have its own controller.

With reference to FIGURES 1, 2 and 3, in one instance the PET examination region 112 and the CT examination region 128 are disposed along a common longitudinal or z-axis 150. In instances in which the first imaging sub-system 104 and the second imaging sub-system 106 are separate imaging systems, each of the sub-systems 104 and 106 will have its own subject support and z-axis. The imaging system 102 further includes an operator console 152 configured to control the imaging system 102. In one instance, the operator console 152 is configured to receive image data at least from the PET DAS 120, the CT DAS 136, the PET reconstructor 122, and/or the CT reconstructor 138. In instances in which the first imaging sub-system 104 and the second imaging sub-system 106 are separate imaging systems, each of the sub-systems 104 and 106 will have its own operator console.

With reference to FIGURE 1, the imaging system 102 is in electrical communication with a data repository(ies) 154. In one instance, the data repository(ies) 154 includes a radiology information system (RIS), a hospital information system (HIS), an electronic medical record (EMR), a picture archiving and communication system (PACS), a server, a database, a cloud-based resource, etc. In one instance, the data repository(ies) 154 is configured to receive PET data and/or a PET image from the imaging system 102 and/or transmit PET data and/or a PET image to the imaging system 102, e.g., via Digital Imaging and Communications in Medicine (DICOM) protocol and/or other protocol.

FIGURE 4 schematically illustrates an example of the operator console 152. The example operator console 152 includes a computing system such as a computer, a workstation, a server, or the like, configured for operating the imaging system 102, including operating the first imaging sub-system 104 and the second imaging sub-system 106.

The operator console 152 includes an input device(s) 402 such as a keyboard, mouse, touchscreen, microphone, etc. In one instance, the input device(s) 402 is configured to receive user input, e.g., selecting a volume of interest, etc. The operator console 152 further includes an output device(s) 404, which includes a human readable device such as a display monitor or the like. In one instance, the output device(s) 404 is configured to display PET data, a PET image, etc. The operator console 152 further includes input/output (I/O) 406 for transmitting and/or receiving signals and/or data.

The operator console 152 further includes a processor(s) 410 such as a micro-processing unit (MPU), a central processing unit (CPU), a graphics processing unit (GPU), etc. The operator console 152 further includes a computer readable storage medium 412, which includes non-transitory medium (e.g., a storage cell, device, etc.) and excludes transitory medium (i.e., signals, carrier waves, and the like). The computer readable storage medium 412 is encoded with computer executable instructions and/or data 414.

In one instance, the data 414 at least includes PET data and/or a PET image, e.g., received or retrieved from the PET DAS 120, the CT DAS 136, the PET reconstructor 122, the CT reconstructor 138, the data repository(ies) 154 of FIGURE 1, and/or other apparatus. In one instance, the data 414 further includes PET data, CT data, a PET image, and/or a CT image generated and/or manipulated by the processor(s) 410.

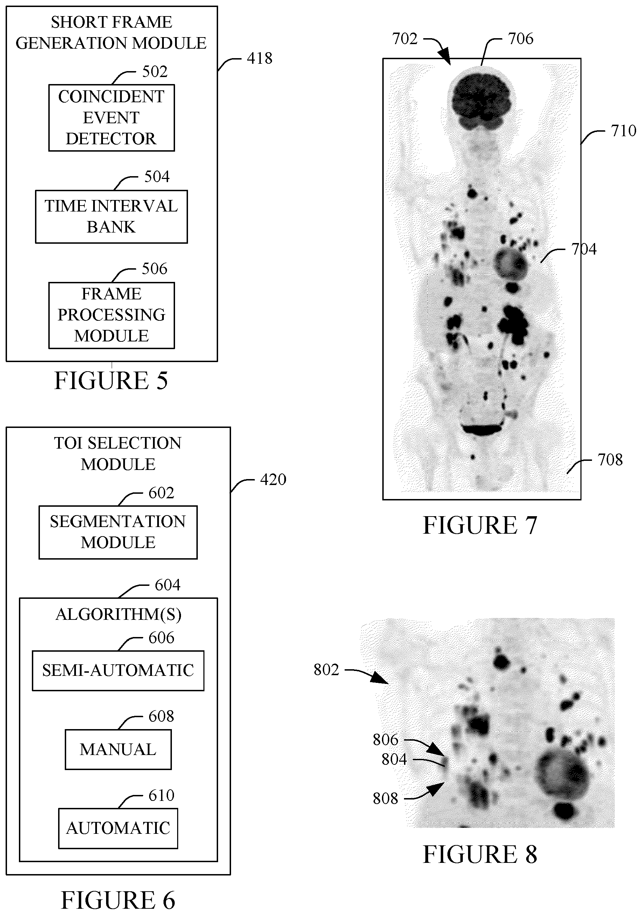

The processor(s) 410 is configured to execute at least one of the computer executable instructions, employ and/or generate the data 414, etc. In one instance, the computing executable instructions include a PET data viewing module 416, a short frame generation module 418, a tissues of interest (TOI) selection module 420, and a motion correction with local patches module 422, which are briefly described next and further described in greater detail in FIGURES 5, 6, 13 and 17.

The PET data viewing module 416 is configured to display PET data, e.g., for selecting tissue of interest, identifying a volume of interest, presenting motion corrected patches, presenting the full PET data with the motion corrected patches, etc. The short frame generation module 418 generally includes instructions for generating a short PET frame(s) based on acquired PET data and a pre-determined time duration. The tissues of interest (TOI) selection module 420 is configured to select at least two tissues of interest respectively within corresponding regions of interest. The motion correction with local patches module 422 is configured to separately and independently estimate motion for the at least two tissues of interest, and separately and independently generate motion corrected patches for the at least two tissues of interest within the corresponding regions of interest.

As described herein, the individual motion corrected patches can be displayed and/or inserted into a rendering of the PET data. Again, the tissue of interest in the local volume of interest has a strong signal that can be readily tracked, whereas tissues in other regions may have weak signals that may not be able to be readily tracked, which means that local regions of interest cannot be placed everywhere, a full FOV of the PET data will further include tissues that are moving differently, and, thus, a rigid motion cannot be assumed, and motion correcting patches instead of the entire PET data will require less resources (e.g., processing power and/or memory) at least since the total number events will be lower than with the full PET data.

FIGURE 5 schematically illustrates an example of the short frame generation module 418. The short frame generation module 418 receives, as input, PET data including data generated by moving tissues of interest. In one instance, the PET data is received and/or retrieved from the PET DAS 120 and/or the data repository(ies) 154 of FIGURE 1, the data 414 of FIGURE 4, and/or other apparatus. In one instance, the short frame generation module 418 receives the PET data after the PET scan and generates the short PET frames from the PET data. In another instance, the short frame generation module 418 receives PET data as the PET data is being acquired during a PET scan and generates the short PET frames as the PET data is received.

The short frame generation module 418 includes a coincident event detector 502. The coincident event detector 502 is configured to identify coincident events in the PET data. For example, the coincident event detector 502 identify a pair of events as a coincident event where the events are detected along a line-of-response (LOR) within a predetermined time of each other to indicate coincidence. Pairs of detected events determined to be coincident events are recorded as coincident events. Events that cannot be paired are discarded.

The short frame generation module 418 includes a time interval bank 504. The time interval bank 504 includes at least one time duration that is used to define time windows over which to generate short PET frames. For example, where the motion cycle is the respiratory cycle, the at least one time duration may be ~0.5 seconds. Where short PET frames are generated for contiguous time windows, a first PET frame is generated with PET data acquired during the first 0.5 seconds of the scan, a next PET frame is generated with PET data acquired during the next 0.5 seconds of the scan, ..., and a last PET frame is generated with PET data acquired during the last 0.5 seconds of the scan.

In another instance, one or more of the time windows may overlap. For example, a first PET frame may be generated with PET data acquired from 0.0 to 0.5 seconds of the scan, a next PET frame may be generated with PET data acquired from 0.4 to 0.9 seconds of the scan, .... In another instance, one or more of the time windows may include a time gap in between with PET data that is not utilized to generate a PET frame. For example, a first PET frame may be generated with PET data acquired from 0.0 to 0.5 seconds of the scan, a next PET frame may be generated with PET image data acquired from 0.6 to 1.0 seconds of the scan, .... Other overlap and/or time gaps are contemplated herein, including a varying overlap and/or time gap, e.g., based on the phase.

In one instance, the time interval is a default value. In one instance, the default value is a constant such as ~0.5 seconds. In another instance, the time interval varies, e.g., where the cyclic motion varies. In another instance, the time interval bank 504 includes multiple time intervals. Such intervals may be based on the tissue of interest, the source of the motion, the imaging center, the radiologist assigned to read the PET data, etc. In another instance, an operator may change or specify the time interval, e.g., from a list of optional time intervals. In another instance, the short frame generation module 418 automatically selects a time interval from a list of optional time intervals based on, e.g., the tissue of interest, the source of the cyclic motion, etc. In another instance, the short frame generation module 418 directly computes the time intervals.

The short frame generation module 418 further includes a frame processing module 506. The frame processing module 506 is configured to generate a short PET frame with the coincident pairs in the bin, for all of the bins in the data buffer and/or other memory. As such, each short PET frame will represent neighboring time windows of the PET acquisition. The frame processing module 506 can employ known iterative and/or other techniques to generate the short PET frames. In one instance, the frame processing module 506 is configured to apply attenuation and/or scatter correction, and/or other signal processing techniques. In another instance, the frame processor 506 does not apply any signal processing techniques.

In another instance the frame processing module 506 generates short PET frames employing the algorithm(s) described in patent US 11,179,128 B2, serial number US 16/732,250, filed on December 31, 2019, and entitled "Methods and Systems for Motion Detection in Positron Emission Tomography," which is incorporated by reference in its entirety herein. In US 11,179,128 B2, a series of live PET frames are generated during a defined time duration while acquiring the emission data. In a variation, the frame processing module 506 employs the algorithm of US 11,179,128 B2 to generate a set of short PET frames after the PET scan from the full acquired PET data and not during acquisition.

FIGURE 6 schematically illustrates an example of the tissues of interest (TOI) selection module 420. The TOI selection module 420 includes a segmentation module 602 and an algorithm(s) 604, including a semi-automatic selection algorithm(s) 606, a manual selection algorithm(s) 608, and/or an automatic selection algorithm(s) 610.

In one instance, for selection of tissues of interest, the PET viewing module 416 of FIGURE 4 displays a rendering of the PET data. FIGURE 7 graphically illustrates an example of a rendering including a Maximum Intensity Projection (MLP) rendering 702. The MLP rendering 702 includes a patient 704 from the head 706 to the thighs 708. In one instance, the MIP rendering 702 is displayed in a viewport 710 of a display monitor of the output device(s) 406. Other renderings (e.g., Minimum Intensity Projection (mIP), etc.) are also contemplated herein.

In the MIP rendering 702, voxels corresponding to projections with higher intensity are darker gray or black and voxels corresponding to projections with lower intensity are lighter gray or white. Higher intensity corresponds to tissues with greater uptake of the radiopharmaceutical and emission of gamma rays. Since a PET scan is acquired over several minutes during periodic and/or non-periodic motion, tissues, including those with radiopharmaceutical uptake, will move with the periodic and/or non-periodic motion, and, as a consequence, tissues with radiopharmaceutical uptake will show up in the MIP rendering 702 blurred.

An example of such blur is illustrated in FIGURE 8, which shows a magnified view of a sub-portion 802 of the MIP rendering 702 of FIGURE 7. In FIGURE 8, the tissue 804 is seen as a streak between a first end 806 and a second end 808. In general, the first end 806 represents an extent of the motion of the tissue 804 in a first visible direction and the second end 808 represents an extent of the motion of the tissue 804 in a second opposing visible direction. The tissue 804 may also move in a direction not visible in FIGURE 8. In FIGURE 8, the movement of the tissue 804 is generally up and down with respect to the sub-portion 802. In another instance, the motion may be left and right, into and out of, and/or a combination of the three directions.

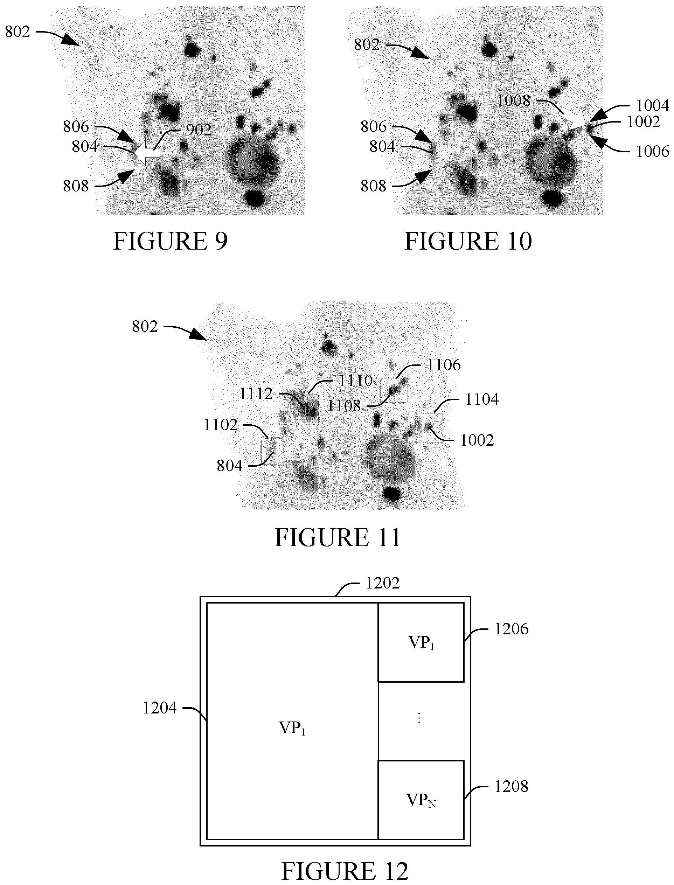

With reference to FIGURES 6, 7 and 8, the TOI selection module 420 receives, as input, a signal from the input device(s) 402 indicative of a user selection on the displayed MIP rendering 702. For example, the user may utilize a mouse (a touchscreen, a stylus, and/or other input device) of the input device(s) 402 to hover over and click on a portion of tissue of interest. An example is shown in FIGURE 9, which shows the sub-portion 802 of FIGURE 8 with a pointer 902 hovering over the burred tissue 804. In one instance, the user clicks the mouse to select the tissue 804.

As described herein, at least two tissues are selected. FIGURE 10 shows a second tissue 1002, which is seen as a streak between a first end 1004 and a second end 1006, where the first end 1004 represents an extent of the motion of the second tissue 1002 in a first direction and the second end 1006 represents an extent of the motion of the second tissue 1002 in a second opposing direction. Likewise, the tissue 1002 may also move in a direction not visible in FIGURE 10. In FIGURE 10, a pointer 1008 hovers over the blurred tissue 1002, and the user clicks the mouse to select the blurred tissue 1002.

Returning to FIGURE 6, the segmentation module 602 segments the selected tissue, e.g., the tissue 804 in FIGURE 9 and/or the tissue 1002 in FIGURE 10. In one instance, the segmentation module 602 segments tissue after each selection, e.g., segments the tissue 804 in FIGURE 9 after selection of the tissue 804, segments the tissue 1002 in FIGURE 10 after selection of the tissue 1002, .... In another instance, multiple tissues are first selected and then the segmentation module 602 segments the multiple tissue, serially and/or in parallel.

Returning to FIGURE 6, the segmentation module 602 segments the selected tissue based on one or more of the algorithm(s) 604. With a non-limiting example semi-automatic selection algorithm(s) 606, after the user selects the tissue of interest (e.g., 804 and/or 1002) the segmentation module 602 segments the selected tissue of interest, utilizing known and/or other segmentation algorithms. Examples of suitable algorithms include histogram-based, edge detection, clustering, thresholding, gradient, volume growing, artificial intelligence-based, and/or other approaches.

The semi-automatic algorithm(s) 606 then automatically places a bounding shape (referred to herein also as a volume of interest, or VOI) around the selected tissue of interest. The bounding shape can be circular, oval, spherical, square, rectangular, cuboid, cylindrical, etc. In one instance, the semi-automatic algorithm estimates a center region (e.g., a center of mass) of the selected tissue and centers the bounding shape about the center. The semi-automatic algorithm estimates an overall size of the selected tissue and generates a bounding shape that is large enough to cover the movement (e.g., overall size + a margin) and small enough so that other tissue in the bounding shape moves approximately with the same displacement.

FIGURE 11 illustrates the sub-portion 802 of FIGURE 8 with several bounding shapes, including a first bounding shape 1102 for the selected tissue 804 of FIGURE 8, a second bounding shape 1104 for the selected tissue 1002 of FIGURE 10, a third bounding shape 1106 for a selected tissue 1108, and a fourth bounding shape 1110 for a selected tissue 1112. The tissues of interest 1108 and/or 1112 can be selected as described above in connection with the tissues of interest 804 and/or 1002, and/or otherwise. Again, at least two tissues of interest are selected, and more than four tissues of interest can be selected.

Returning to FIGURE 6, in one instance, in addition to the displaying the sub-portion 802 with bounding shapes (e.g., FIGURE 11), the PET data viewing module 416 of FIGURE 4 can display one or more other viewports (e.g., other than the viewport 710 of FIGURE 7) with one or more planes of the PET data that includes the selected tissue. Examples of such planes are axial, sagittal, coronal, oblique, irregular, etc. planes. For example, in one instance the PET data viewing module 416 presents four viewports, one with the MIP 702 or the sub-portion 802, one with an axial slice, one with a sagittal slice, and one with a coronal slice.

FIGURE 12 schematically illustrates a GUI 1202 with N viewports, a viewport 1 (VPi) 1204, ..., , a viewport I (VP_{I}) 1206, ..., and a viewport N (VP_{N}) 1208, where N is a positive integer greater than two. In the illustrated embodiment, the viewport 1 and the set of viewports I-N are positioned horizontally with respect to each other, and the viewports I through N are positioned vertically with respect to each other. In addition, the viewport 1 is shown larger than the viewports I and N. This illustration is non-limiting and other configurations are contemplated herein. In addition, a user can toggle the visibility of one or more of the N viewports on and off.

In one instance, the VOI is also presented in each plane. The user, via an input device of the input device(s) 402, can accept, reject and/or modify the automatically generated and placed VOI in one of more dimensions. In one instance, a VOI further includes a mask or sub-VOI within the VOI. In this instance, the VOI can be set larger and the mask or sub-VOI can be moved within the larger VOI over the tissue of interest, e.g., during motion tracking. In general, the tissue of interest will move in a known direction and in small increments from frame to frame so the mask or sub-VOI can be automatically moved for a frame based on its location for the previous frame, which will provide a more accurate motion measurement.

With a non-limiting example manual selection algorithm(s) 608, the TOI selection module 420 provides soft tools for manual selection of tissues of interest. In one instance, the soft tools are provided via a drop-down menu, a pop-up menu, a list box, a directory tree structure, etc. In this instance, the user selects a bounding shape from the menu (e.g., a rectangle, etc.), and the TOI selection module 420 displays a bounding shape which the user can move around the viewport, change its shape, and fix it to a point in the viewport.

Similar to the above-discussed semi-automatic selection algorithm(s) 606, the bounding box can be sized and placed on the MIP rendering 702 of FIGURE 8 and/or the sub-portion 802 of FIGURE 8, and/or with one or more views displayed in one or more viewports. Likewise, the user, via an input device of the input device(s) 402, can accept, reject and/or modify the VOI in one of more dimensions. In one instance, the above-discussed semi-automatic algorithm(s) 606 is utilized to select a tissue of interest, and the subject manual algorithm(s) 608 is utilized to select another tissue of interest. In another example, the manual tool is a free hand drawing tool.

With a non-limiting example automatic selection algorithm(s) 610, the TOI selection module 420, the algorithm identifies the at least two tissues of interest and generates the VOIS. This may include automatically identifying the tissues of interest such as a tumor, a lesion, an organ, etc., based on information with the PET data file such as a header, metadata, a DICOM field, etc. In one instance, the algorithm includes a deep learning (DL) based algorithm trained to identify organs and/or anomalies in normal tissue. In a longitudinal study, a VOI in a previous image of a patient can be used to identify the tissue of interest in a current image of the patient by mapping the VOI between the images.

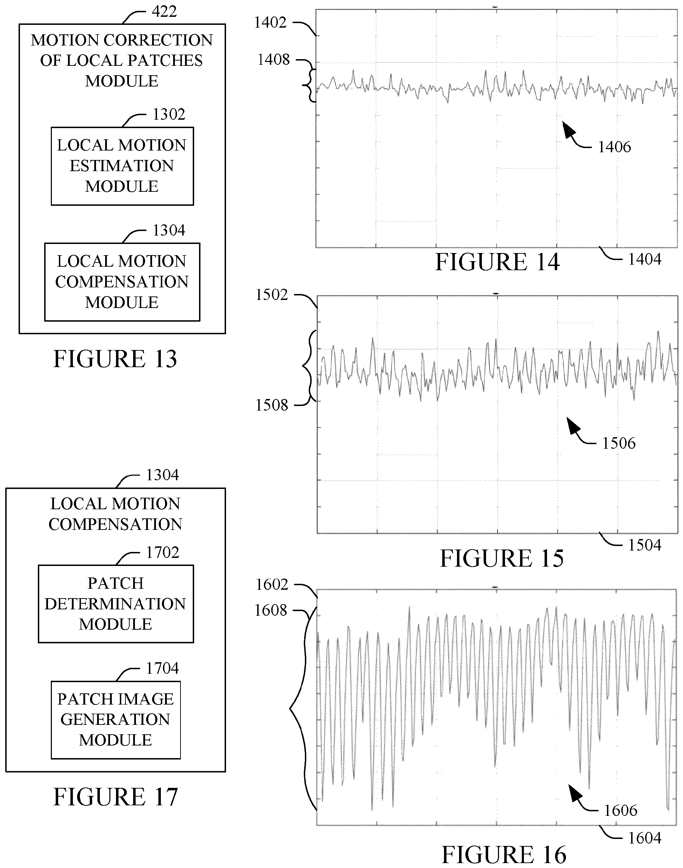

FIGURE 13 schematically illustrates an example of the motion correction with local patches module 422. The example motion correction with local patches module 422 includes a local motion estimation module 1302 and a local motion compensation module 1304. The motion correction with local patches module 422 obtains, as input, the set of short PET frames generated by the frame processing module 506 of the short frame generation module 418 and/or other processor. The set of short PET frames can be obtained (e.g., received and/or retrieved) from the data 414, the repository(ies) 154, and/or other apparatus.

The location motion estimation module 1302 separately and independently estimates a motion of the tissues of interest in each VOI and/or mask/sub-VOI. The location motion estimation module 1302 estimates the motion in the different each VOIS and/or masks/sub-VOIS in series or parallel. In the discussion below, the location motion estimation module 1302 estimates the motion based on the short PET frames. Additionally, or alternatively, the location motion estimation module 1302 estimates the motion based on time-of-flight (TOF) point-cloud representations of the data, backprojections, center-of-mass calculations from the list-mode, sinogram data, and/or other information.

The local motion estimation module 1302, for selected TOI and a corresponding VOI, tracks the motion of the TOI through the short PET frame. In one instance, local motion estimation module 1302 defines a spherical region of constant radius (e.g., 0.5 cm 1 cm, 2 cm, 5 cm, etc.) centered on a previous location of the target. For a first motion point, the provided target location can be used to center the spherical region. Non-limiting examples of suitable approaches to estimate the location of the target include calculating a center-of-mass, registering an image to a chosen reference frame, locating a maximum pixel, etc. In one embodiment, one or more of these approaches and/or other approach can be combined with image pre-processing steps such as smoothing, thresholding, etc.

In general, the center-of-mass is a weighted mean of the pixels and can be computed, e.g., by summing the product of each pixel's value and position, and dividing the result by a sum of the values. In one instance, the pixel values in the VOI are first thresholded, e.g., by subtracting a fraction of a maximum pixel value from all pixel values and then setting all negative pixel values to zero. The center-of-mass provides an estimate of the center of the target in each short PET frame. The center-of-mass of the short PET frames can then be tracked through the short PET frames to track the motion of the tissue of interest. Another non-limiting approach includes registering the frames to determine a displacement of the identified tissue of interest from frame to frame. Other approaches are contemplated herein.

Where a mask/sub-VOI is utilized, the location motion estimation module 1302 masks out all pixels inside of the VOI that are outside of the mask/sub-VOI. The pixel values in a mask/sub-VOI can first be thresholded, as discussed herein and/or otherwise. As a result, pixel values at the edge of a mask/sub-VOI are zero or close to zero. For each TOI, the local motion estimation module 1302 determines a motion within the spherical volume of constant radius. In one instance, the local motion estimation module 1302 estimates motion with at least one degree of freedom, e.g., at least one of translation in the x direction, rotation in the x direction, translation in the y direction, rotation in the y direction, translation in the z direction, and rotation in the z direction, a Cartesian coordinate system. In another instance, the local motion estimation module 1302 estimates motion with two, three, four, five or six degrees of freedom.

FIGURES 14, 15 and 16 respectively graphically show estimated motion in the x direction, the y direction, and the z direction for the TOI 804 in the VOI 1102 in FIGURE 11. Beginning with FIGURE 14, a first axis 1402 represents position, e.g., in millimeters or other units. A second axis 1404 represents time, e.g., in seconds or other units. A plot 1406 represents position versus time in the x direction. In general, the time points of the second axis 1404 are based on the time window of the short PET frames, e.g., 0.5 s increments. Interpolation and/or other processing can be used to derive position data between the time points.

In FIGURE 15, a first axis 1502 represents position, e.g., in millimeters or other units. A second axis 1504 represents time, e.g., in seconds or other units. A plot 1506 represents position versus time in the y direction. In FIGURE 16, a first axis 1602 represents position, e.g., in millimeters or other units. A second axis 1604 represents time, e.g., in seconds or other units. A plot 1606 represents position versus time in the z direction. Likewise, the time points of the second axes 1504 and 1604 are determined by the time window of the short PET frames, e.g., 0.5 s increments, and interpolation and/or other processing can be used to derive position data between the time points.

From FIGURES 14, 15 and 16, a least amount motion of the TOI 804 due to period and/or non-period motion is in the x direction, as an amplitude of variation 1408 of the plot 1406 is less than in an amplitude of variation 1508 of the plot 1506 and an amplitude of variation 1608 of the plot 1606. A greatest amount motion of the TOI 804 due to period and/or non-period motion is in the z direction, as the amplitude of variation 1608 of the plot 1606 is greater than in the amplitude of variation 1508 of the plot 1506 and the amplitude of variation 1408 of the plot 1406. Again, rotation and/or other movement can also be tracked.

If it is determined that a VOI for a tissue of interest is not large enough for a short PET frame and the tissue of interest in the VOI moves outside of its VOI for the short PET frame, the VOI can be adjusted for the short PET frame in real-time based on estimated motion, e.g., and increased by a predetermined amount. This determination can be made based on the known location of the edge or perimeter of the VOI and the estimated motion. Since motion is tracked for each VOI, this determination can be made for each VOI for all short PET frames.

Returning to FIGURE 13, the location motion estimation module 1302 estimates the motion of tissue of interest for at least one other VOI (i.e., at least two in total). As discussed herein, the local motion estimation module 1302 estimates the motion of the TOIS in the VOIS separately and independently, in series or parallel. That is, the local motion estimation module 1302 estimates the motion of the tissue of interest 804 in FIGURE 11 based on events in the VOI 1102 of FIGURE 11, estimates the motion of the tissue of interest 1002 in FIGURE 11 based on events in the VOI 1104 of FIGURE 11, etc.

FIGURE 17 schematically illustrates a non-limiting example of the local motion compensation module 1304. The local motion compensation module 1304 includes a patch determination module 1702 and a patch image generation module 1704. The patch determination module 1702 determines patches for the VOIS. In one instance, a size of a patch for TOI in the VOI is based on the estimated motion. In one instance, the size of a patch is the same as the VOI. In another instance, the size of the patch is smaller than the size of the corresponding VOI, e.g., where it is determined that the patch size can be decreased and all of the motion can still be corrected based on the estimated motion. Similar to the VOI, the patch can be variously shaped, e.g., rectangular, spherical, cylindrical, etc.

The patch image generation module 1704 separately and independently processes the patches. In one instance, only events in a patch are processed to correct the events in the patch for the estimated motion. In one instance, the patch image generation module 1704 account for activity outside of the patch during processing. For list-mode processing, this can be accomplished by forward projecting through a full field of view (FOV) image. The FOV image can be low fidelity and, thus, a low resolution image without motion correction can be utilized. The forward projection of each event through the full FOV image can then be added to the forward projection through the patched reconstruction during each image update. Accounting for activity outside of the patch can ensure quantitative accuracy.

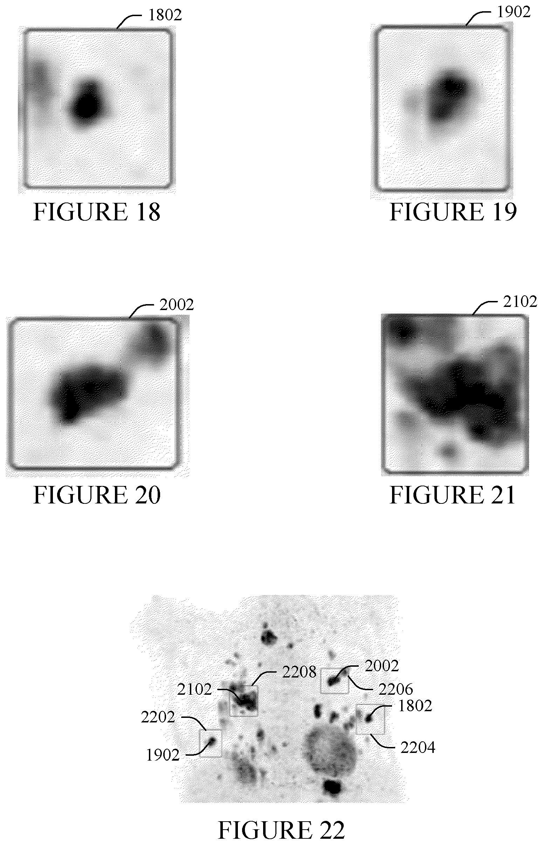

As discussed herein, the motion corrected patches can be displayed and/or inserted into the MIP rendering 702 of FIGURE 7 and/or the sub-portion 802 in FIGURE 8. FIGURES 18, 19, 20 and 21 show individual motion corrected patches 1802, 1902, 2002, and 2102 respectively for the tissues of interest 804, 1002, 1108 and 1112 in FIGURE 11. The tissues of interest in the patches 1802, 1902, 2002, and 2102 are sharper and include less blur than the corresponding tissues of interest 804, 1002, 1108 and 1112 in FIGURE 11 that is not corrected. As such, the patches 1802, 1902, 2002, and 2102 more accurately capture the activity and distribution in the tissues of interest.

FIGURE 22 shows the patches 1802, 1902, 2002, and 2102 inserted into the sub-portion 802 in FIGURE 8. In FIGURE 22, visible borders 2202, 2204, 2206, and 2208 respectively are provided around the patches 1802, 1902, 2002, and 2102 at least so that the observer knows which portion of the sub-portion 802 is corrected for motion and which portion of the sub-portion has not been corrected for motion. Inserting the patches 1802, 1902, 2002, and 2102 inserted into the sub-portion 802 in FIGURE 8 allows for visualizing the motion corrected tissues of interest in context with patient anatomy.

In another instance, averaging, smoothing, etc. can be utilized to seam the patches into sub-portion 802 for a more natural appearance. In another instance, the patch image generation module 1704 motion corrects the full PET data. In this instance, the patches 1802, 1902, 2002, and 2102 are segmented or cropped from the motion corrected full PET data and displayed as described herein, e.g., individually as shown in FIGURES 18-21 and/or inserted into the sub-portion 802 as shown in FIGURE 22 or the MIP rendering 702 of FIGURE 7. However, processing only the patches requires less processing resources and/or consumes less time relative to processing the entire full PET data.

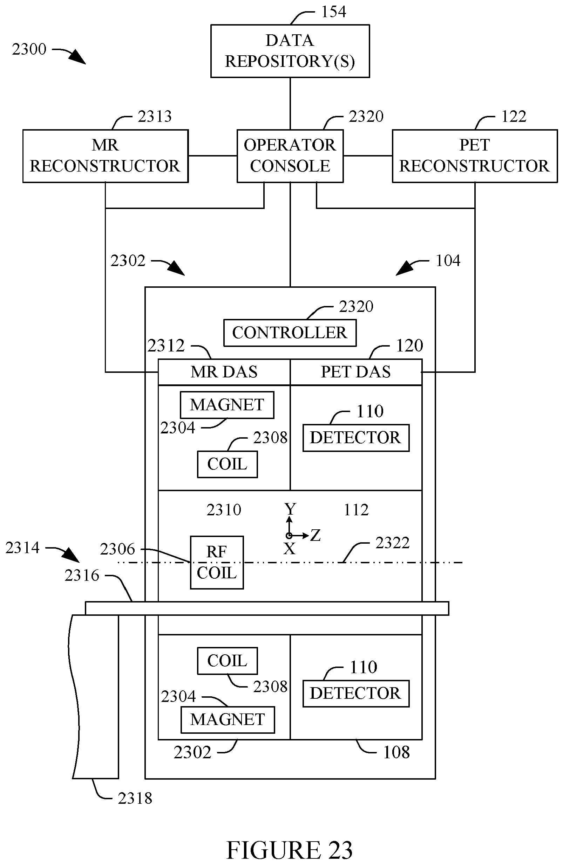

In FIGURE 1, the imaging system is a PET/CT hybrid imaging system. FIGURE 23 schematically illustrates a variation 2300 of FIGURE 1 in which the imaging system 102 includes the PET imaging sub-system 104 and an MR imaging sub-system 2302. The MR imaging sub-system 2302 includes a main magnet 2304, a gradient (x, y, and z) coil(s) 2308, and a RF coil 2306. The main magnet 2304 (which can be a superconducting, resistive, permanent, or other type of magnet) produces a substantially homogeneous, temporally constant main magnetic field B0 in an MR examination region 2310. The gradient coil(s) 2308 generate time varying gradient magnetic fields along the x, y, and z-axes of the MR examination region 2310.

The RF coil 2306 includes a transmit portion that produces radio frequency signals (at the Larmor frequency of nuclei of interest (e.g., hydrogen, etc.)) that excite the nuclei of interest in the examination region 2310 and a receive portion that detects MR signals emitted by the excited nuclei. In other embodiments, the transmit portion and the receive portion of the RF coil 2306 are located in separate RF coils 2306. A MR data acquisition system (DAS) 2312 processes the MR signals, and a MR reconstructor 2313 reconstructs the data and generates MR images.

A subject support 2314 includes a tabletop 2316 moveably coupled to a frame/base 2318. In one instance, the tabletop 2316 is slidably coupled to the frame/base 2318 via a bearing or the like, and a drive system (not visible) including a controller, a motor, a lead screw, and a nut (or other drive system) translates the tabletop 2316 along the frame/base 2318 into and out of the examination region 2310 and/or 112. The tabletop 2416 is configured to support an object or subject in the examination region 2310 and/or 112 for loading, scanning, and/or unloading the subject or object.

A controller 2320 is configured to control components such as the main magnet 2304, the gradient coil(s) 2308, the RF coil 2306, an operation of the detector array 110, an operation of the subject support 2314, etc. The PET examination region 112 and the MR examination region 2310 are disposed along a common longitudinal or z-axis 2322. The imaging system 2300 further includes an operator console 2320, which is substantially similar to the operator console 152 of FIGURE 1 (except that it is configured for the MR imaging sub-system 2302 instead of the CT imaging sub-system 106) and, as such, is not described again. In instances in which the imaging sub-systems 104 and 2302 are separate imaging systems, each of the sub-systems 104 and 106 will have its own controller, subject support, and operator console.

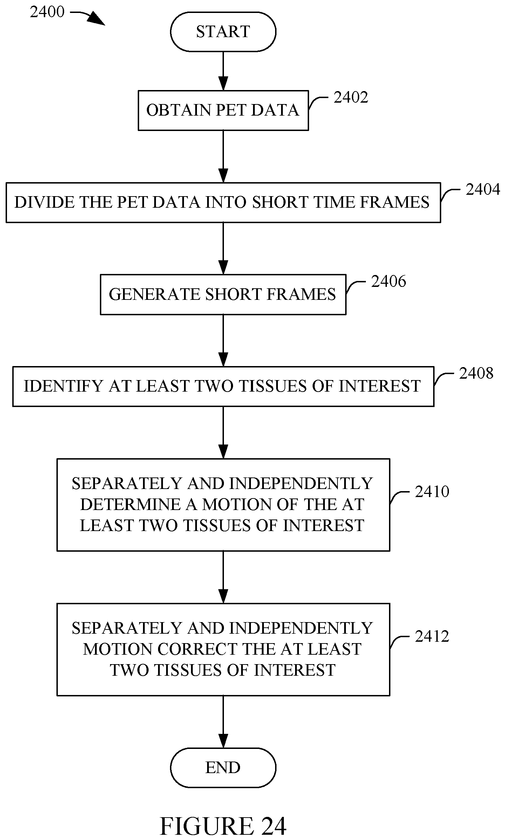

FIGURE 24 illustrates a non-limiting example of a flow chart for a computer-implemented method. It is to be appreciated that the ordering of the acts in the method is not limiting. As such, other orderings are contemplated herein. In addition, one or more acts may be omitted, and/or one or more additional acts may be included.

At 2402, PET data of moving tissue of interest is obtained, as described herein and/or otherwise. At 2404, the PET data is divided into a plurality of short time frames based on a predetermined time duration, as described herein and/or otherwise. At 2406, the divided PET data is processed to generate a set of short PET frames, as described herein and/or otherwise. At 2408, at least two tissues of interest are identified in the PET data, as described herein and/or otherwise.

At 2410, the motion of each of the at least two tissues of interest is separately and independently estimated with the set of short PET frames, as described herein and/or otherwise. At 2412, the motion of each of the at least two tissues of interest is separately and independently corrected based on the estimated motion, as described herein and/or otherwise. As discussed herein, the motion corrected at least two tissues of interest can be displayed and/or inserted into a PET data rendering.

The above method(s) can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include such additional elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), logic circuits, and any other circuit or processor capable of executing the functions described herein. The above examples are exemplary only, and are thus not intended to limit in any way the definition and/or meaning of the term "computer". The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present disclosure. That is, features of the described embodiments can be combined with any appropriate aspect described above and optional features of any one aspect can be combined with any other appropriate aspects. Similarly, features set forth in dependent claims can be combined with non-mutually exclusive features of other dependent claims, particularly where the dependent claims depend on the same independent claim. Single claim dependencies may have been used as practice in some jurisdictions that require them, but this should not be taken to mean that the features in the dependent claims are mutually exclusive.

## Claims

1. A computer-implemented method, comprising:
obtaining positron emission tomography (PET) data that includes moving tissue of interest;
generating a set of short PET frames from the PET data, wherein each short PET frame of the set of short PET frames is based on a time duration;
identifying a first tissue of interest in the set of short PET frames;
identifying at least a second tissue of interest in the set of short PET frames;
estimating, separately and independently, a first motion of the first tissue of interest and a second motion of second tissue of interest based on the short PET frames; and
motion correcting, separately and independently, the first tissue of interest for the first motion and the second tissue of interest for the second motion.

2. The computer-implemented method of claim 1, further comprising:
displaying the motion corrected first tissue of interest and the second motion corrected second tissue of interest.

3. The computer-implemented method of claim 1, further comprising:
inserting the motion corrected first tissue of interest at a corresponding first location in a rendering of the PET data;
inserting the motion corrected second tissue of interest at a corresponding second location in the rendering of the PET data; and
displaying the rendering of the PET data.

4. The computer-implemented method of claim 1, wherein at least one of the first motion and the second motion includes at least one of a translation in an x direction, a rotation about the x direction, a translation in a y direction, a rotation about the y direction, a translation in a z direction, and a rotation about the z direction.

5. The computer-implemented method of claim 1, further comprising:
generating a first volume of interest for the first tissue of interest, wherein the first volume of interest is less than an entire field of view of the PET data and only PET data within the first volume of interest is utilized to estimate the first motion; and
generating a second volume of interest for the second tissue of interest, wherein the second volume of interest is less than the entire field of view of the PET data and only PET data within the second volume of interest is utilized to estimate the second motion,
wherein the first volume of interest and the second volume of interest are different volumes of interest.

6. The computer-implemented method of claim 5, further comprising:
determining a first patch within the first volume of interest to motion correct; and
determining a second patch within the second volume of interest to motion correct.

7. The computer-implemented method of claim 6, wherein the first volume of interest has a first size, the second volume of interest has a second size, the first patch has a third size, and the second patch has a fourth size, and the third size is equal to the first size and the fourth size is equal to the second size.

8. The computer-implemented method of claim 6, wherein the first volume of interest has a first size, the second volume of interest has a second size, the first patch has a third size, and the second patch has a fourth size, and the third size is smaller than the first size and the fourth size is smaller than the second size.

9. The computer-implemented method of claim 5, further comprising:
modifying at least one of a first size, a first location, or the first size and the first location of the first volume of interest and a second size, a second location, or the second size and the second location of the second volume of interest for a short PET frame during motion estimation based on the estimated motion.

10. The computer-implemented method of claim 1, wherein at least one of the first motion and the second motion is rigid motion.

11. A computing system (154), comprising:
a computer readable storage medium (412) that includes instructions for correcting motion in data; and
a processor (410) configured to execute the instructions, wherein the instructions cause the processor to:
obtain PET data that includes moving tissue of interest;
generate a set of short PET frames from the PET data, wherein each short PET frame of the set of short PET frames is based on a time duration;
identify a first tissue of interest in the set of short PET frames;
identify at least a second tissue of interest in set of short PET frames;
estimate, separately and independently, a first motion of the first tissue of interest and a second motion of second tissue of interest based on the short PET frames; and
motion correct, separately and independently, the first tissue of interest for the first motion and the second tissue of interest for the second motion.

12. The computing system of claim 11, wherein the instructions cause the processor to:
display the motion corrected first tissue of interest and the second motion corrected second tissue of interest.

13. The computing system of claim 11, where the instructions further cause the processor to:
insert the motion corrected first tissue of interest at a corresponding first location in a rendering of the PET data;
insert the motion corrected second tissue of interest at a corresponding second location in the rendering of the PET data; and
display the rendering of the PET data.

14. The computing system of claim 11, wherein at least one of the first motion and the second motion includes at least one of a translation in an x direction, a rotation about the x direction, a translation in an y direction, a rotation about the y direction, a translation in a z direction, and a rotation about the z direction.

15. A computer readable storage medium encoded with computer executable instructions, which when executed by a processor, causes the processor to:
obtain PET data that includes moving tissue of interest;
generate a set of short PET frames from the PET data, wherein each short PET frame of the set of short PET frames is based on a time duration;
identify a first tissue of interest in the set of short PET frames;
identify at least a second tissue of interest in the set of short PET frames;
estimate, separately and independently, a first motion of the first tissue of interest and a second motion of second tissue of interest based on the short PET frames; and motion correct, separately and independently, the first tissue of interest for the first motion and the second tissue of interest for the second motion.
